# EUROPÄISCHE PATENTSCHRIFT

(11) **EP 3 702 755 B1**
(45) Veröffentlichungstag und Bekanntmachung des Hinweises auf die Patenterteilung: **29.07.2026**
(21) Anmeldenummer: 20163624.8
(22) Anmeldetag: 26.10.2015
(51) Int. Cl.: G01N 21/21

(54) **DETEKTOR ZUR ERFASSUNG OPTISCHER STRAHLUNG**
DETECTOR FOR DETECTING OPTICAL RADIATION
DÉTECTEUR PERMETTANT DE DÉTECTER UN RAYONNEMENT OPTIQUE

(43) Veröffentlichungstag der Anmeldung: 02.09.2020
(62) Teilanmeldung aus: 15787469.4
(73) Patentinhaber: Fraunhofer-Gesellschaft zur Förderung der angewandten Forschung e.V., 80686 München (DE)
(72) Erfinder: Zimmermann, Heiko, 97295 Waldbrunn (DE); Stracke, Frank, 66123 Saarbrücken (DE); Le Harzic, Ronan, 67260 Herbitzheim (FR)
(74) Vertreter: v. Bezold & Partner Patentanwälte - PartG mbB

(56) Entgegenhaltungen:
- WO-A1-2014/100378
- WO-A1-2014/123156
- POMARICO J ET AL: "Compact device for assessment of microorganism motility", REVIEW OF SCIENTIFIC INSTRUMENTS, AIP, MELVILLE, NY, US, vol. 75, no. 11, 1 November 2004 (2004-11-01), pages 4727 - 4731, XP012071874, ISSN: 0034-6748, DOI: 10.1063/1.1809266
- J A COLE ET AL: "Laser speckle spectroscopy-a new method for using small swimming organisms as biomonitors", BIOIMAGING, vol. 4, no. 4, 1 December 1996 (1996-12-01), GB, pages 243 - 253, XP055277248, ISSN: 0966-9051, DOI: 10.1002/1361-6374(199612)4:4<243::AID-BIO3>3.3.CO;2-5

## Beschreibung

Die Erfindung betrifft ein Verfahren und eine Vorrichtung zur optischen In-Vitro-Detektion einer Bewegung in einer biologischen Probe und/oder zur optischen In-Vitro-Detektion einer Bewegung eines Probenbestandteils der biologischen Probe, wobei der Durchmesser der biologischen Probe vorzugsweise mindestens 100 µm beträgt.

Aus der Praxis ist bekannt, dass ein Bedarf an einer Überwachung der aktiven Dynamik von geschlossenen und dreidimensionalen Zell- und Gewebekulturen in Bereichen wie der Entwicklungsbiologie, der Toxizitätstests und der pharmazeutischen Forschung besteht.

Beispielsweise werden aus embryonalen Stammzellen gezüchtete Gewebestücke, die sich zu einem Muskelgewebe differenziert haben, im Rahmen von Toxizitätstests genutzt, um die Schädlichkeit einer zu prüfenden Substanz zu testen. Hierbei wird untersucht, ob eine auf das Muskelgewebe aufgebrachte Substanz die Muskelkontraktionen des Muskelgewebes beeinflusst, was ein Indikator für die Toxizität der Substanz sein kann. Hierfür sind Messverfahren erforderlich, um eine Bewegung, z. B. eine Kontraktion, in einer solchen dreidimensionalen biologischen Probe in Form eines Zellhaufens zu detektieren. Typische Durchmesser solcher Zellhaufen betragen 50 bis 400 µm, wobei auch Durchmesser im Millimeterbereich möglich sind.

Diese Studien werden derzeit in erster Linie durch visuelle Beobachtungen und selten durch Videomikroskopie mit anschließender Bildanalyse durchgeführt. Erstere sind zeitaufwändig und immer mit subjektiver Einschätzung verbunden. Letztere hat den Nachteil, dass komplexe abbildende Optiken und eine komplexe Bildanalyse verbunden mit einem erheblichen Rechenaufwand erforderlich sind. Nachteilig ist ferner ihre inhärente Empfindlichkeit gegenüber geringfügigen Verschiebungen.

Nichtoptische Methoden wie Impedanzmessungen funktionieren nur im Kontakt zur Probe. Wenn die Probeform jedoch von der Ebene (adhärente Monolage) abweicht oder gar dreidimensional ist, noch dazu frei in einem Medium schwimmt, sind die vorgenannten Techniken nicht anwendbar.

Automatisierte bildgebende Verfahren haben den Nachteil, dass z. B. bei einer Schärfentiefe von z.B. 10 µm und der vorstehend genannten typischen Größe der Zellhaufen von 50 bis 400 µm 5 bis 40 Bildebenen der Probe durchgemessen werden müssten. Bei einer typischen Mindestmessdauer von ca. 10 Sekunden, um eine Bewegung detektieren zu können, und der zusätzlich für die Neupositionierung bzw. Fokussierung benötigten Zeit sind derartige Verfahren nicht geeignet, schnell eine Vielzahl von Proben zu überwachen.

Eine serielle Messung von großen Probenzahlen ist aufgrund der durch die Zeitskalen der biologischen Dynamik recht langen Beobachtungsdauer generell nicht angezeigt. In der Praxis besteht jedoch der Bedarf, eine derartige Bewegungsdetektion an einer Vielzahl voneinander getrennter, z. B. in einer Multiwell-Platte, auch als Mikrotiterplatte bezeichnet, z. B. mit in 96 oder 384 Kavitäten (engl. Wells) gelagerten Proben, durchzuführen. Bildgebende Verfahren sind für eine parallele Messung einer Vielzahl solcher Proben nicht geeignet, da es aus geometrischen und bauraumtechnischen Gründen schwierig zu realisieren ist, an jeder Kavität der Multiwell-Platte eine herkömmliche bildgebende optische Vorrichtung anzuordnen.

Aus der Veröffentlichung Pomarico J et al: "Compact device for assessment of microorganism motility", Review of Scientific Instruments, AIP, Melville, NY, US, Bd. 75, Nr. 11, (2005-11-01), Seiten 4727-4731) ist ein Messsystem zur Erkennung einer Motilität von Mikroorganismen bekannt. Der Ansatz basiert auf dynamischer Speckle-Interferometrie, wobei ein Speckle-Muster mittels einer CCD-Kamera aufgezeichnet und anschließend mittels eines PC und einer Bildverarbeitungssoftware ausgewertet wird.

Es ist somit eine Aufgabe der Erfindung, ein verbessertes Verfahren zur Detektion einer Bewegung in einer biologischen Probe und/oder einer Bewegung eines Probenbestandteils der biologischen Probe bereitzustellen, mit dem Nachteile herkömmlicher Techniken vermieden werden können. Der Erfindung liegt insbesondere die Aufgabe zugrunde, ein robustes, kontaktfreies Verfahren zur Bewegungsdetektion bereitzustellen, das keine komplexe Bildanalyse erfordert. Es ist eine weitere Aufgabe der Erfindung, ein Verfahren bereitzustellen, das sich für die parallele Analyse einer Vielzahl von Proben in einer Screening-Umgebung eignet. Eine weitere Aufgabe ist es, eine Vorrichtung zur Detektion einer Bewegung in einer biologischen Probe mit räumlicher Ausdehnung bereitzustellen, mit der Nachteile herkömmlicher Vorrichtungen vermieden werden können.

Diese Aufgaben werden durch Vorrichtungen und Verfahren mit den Merkmalen der unabhängigen Ansprüche gelöst. Vorteilhafte Ausführungsformen und Anwendungen der Erfindung ergeben sich aus den abhängigen Ansprüchen und werden in der folgenden Beschreibung unter teilweiser Bezugnahme auf die Figuren näher erläutert.

Gemäß einem ersten Gesichtspunkt der Erfindung wird ein Verfahren zur optischen In-Vitro-Detektion einer Bewegung in einer biologischen Probe und/oder einer Bewegung eines Probenbestandteils der biologischen Probe bereitgestellt. Bei der biologischen Probe handelt es sich um eine biologische Probe mit räumlicher Ausdehnung. Die biologische Probe ist vorzugsweise eine biologische Probe, die eine Mehrzahl, insbesondere Vielzahl, von biologischen Zellen umfasst. Der Durchmesser der biologischen Probe kann mindestens 50 Mikrometer (µm) in mindestens einer Raumrichtung, weiter vorzugsweise in allen Raumrichtungen betragen und beträgt oft mehr als 100 µm in alle Raumrichtungen. Eine weitere Variante sieht vor, dass ein Durchmesser der biologischen Probe größer ist als eine von der optischen Weitfeldbeleuchtungseinrichtung erreichbare, beugungsbegrenzte Auflösung. Die Erfindung ist jedoch nicht auf biologische Proben mit derartigen Abmessungen beschränkt.

Bei der biologischen Probe kann es sich um eine dreidimensionale Zell- und/oder Gewebekultur handeln, z. B. in Form eines Zellhaufens. Die biologische Probe kann insbesondere lebende Zellen, beispielsweise Muskelzellen, umfassen oder allgemein Zellen, die eine Aktivdynamik aufweisen und/oder eine Bewegung auslösen können. Unter der Detektion einer Bewegung in der biologischen Probe soll insbesondere eine Bewegung innerhalb der Probe oder eine Bewegung eines Probenbestandteils der biologischen Probe verstanden werden. Mit anderen Worten sollen allgemein dynamische Phänomene in bzw. innerhalb biologischer Proben mit räumlicher Ausdehnung detektiert werden können. Bei Zellkulturen aus Muskelzellen können derartige Bewegungen beispielsweise durch Kontraktion einzelner Muskelzellen ausgelöst werden.

Bei der biologischen Probe kann es sich jedoch auch um eine Probe aus freischwimmenden Mikroorganismen, beispielsweise Spermien, handeln. In diesem Fall kann das Verfahren zur Detektion der Bewegung der freischwimmenden Mikroorganismen genutzt werden, beispielsweise im Falle von Spermien zur Bestimmung der Spermienmotilität.

Die biologische Probe wird nachfolgend auch kurz als Probe bezeichnet. Vorzugsweise beträgt der Durchmesser der Probe höchstens 5 mm, weiter vorzugsweise höchstens 1 mm.

Das erfindungsgemäße Verfahren umfasst das Bereitstellen einer optischen Weitfeld-Beleuchtungseinrichtung zur Beleuchtung der Probe, die ausgebildet ist, die ganze Probe zu beleuchten, und ferner das Bereitstellen eines Detektors zur Erfassung der von der Probe kommenden Strahlung.

Hierbei weist der Detektor eine Detektionsfläche auf, die in mehrere Detektionsbereiche unterteilt ist. Beispielsweise kann der Detektor ein zweidimensionaler Pixelarray-Detektor mit matrixartig angeordneten Pixelelementen sein, z. B. ein optischer Pixelsensor. Gemäß einer Variante der Erfindung kann die Detektionsfläche in mindestens 10*10 oder mindestens 100 Detektionsbereiche unterteilt sein. Die Detektionsfläche des Detektors kann in z.B. 10.000 oder mehr mehrzeilig nebeneinander angeordnete Detektionsbereiche, z. B. Pixel, unterteilt sein, z. B. in 100*100 Pixel bzw. Kanäle oder mehr unterteilt sein. Je höher die Ortsauflösung bzw. die Pixelanzahl ist, desto zuverlässiger kann sichergestellt werden, dass sich bewegungsinduzierte Änderungen der erfassten Intensität nicht ausmitteln, sondern kumuliert erfasst werden können.

Die Vorrichtung, insbesondere der Detektor, ist ausgebildet, Detektionssignale einzelner Detektionsbereiche nach der Zeit abzuleiten, anschließend gleichzurichten, wobei das Gleichrichten vorzugsweise durch Betragsbildung oder Quadrierung erfolgt, und die abgeleiteten und gleichgerichteten Detektionssignale aller Detektionsbereiche zu summieren oder zu mitteln. Die Detektionssignale einzelner Detektionsbereiche nach der Zeit abzuleiten bedeutet, dass jeweils die n-te Ableitung, n>=1, nach der Zeit des Detektionssignals jedes Detektionsbereichs, z. B. jedes Pixels, bestimmt wird. Vorzugsweise wird jeweils die erste zeitliche Ableitung bestimmt, d. h. n=1.

Erfindungsgemäß wird somit ein Detektor mit ortsauflösender Detektionsfläche in Form der einzelnen Detektionsbereiche verwendet, die nachfolgend auch als Kanäle oder Pixel bezeichnet werden. Die Messsignale bzw. Messkurven der einzelnen Detektionsbereiche können in digitaler oder analoger Form vorliegen und werden in einem ersten Schritt nach der Zeit differenziert und dann gleichgerichtet. Anschließend werden die gleichgerichteten Signale bzw. Daten der einzelnen Detektionsbereiche jedoch summiert oder gemittelt und ergeben so ein quantitatives Maß der Dynamik in einem von der Probe auf der Detektionsfläche erzeugten Muster oder Bild. Nach außen erscheint der flächenkumulierte Bewegungssensor somit als Detektor, der ein nicht ortsaufgelöstes Signal oder nicht ortsaufgelöste Daten ausgibt. Der Detektor kann somit so ausgeführt sein, dass er ein einkanaliges Signal ausgibt. Aufwändige Analyse wie Mustererkennung und Trajektorienbildung zur Messung und Beschreibung dynamischer Parameter entfallen.

Der Detektor ist somit vorzugsweise ein nicht bildgebender Detektor. Das Ausgabesignal des Detektors kann analog oder digital sein.

Ein weiterer Vorzug besteht darin, dass dynamische Phänomene in der Probe durch Helligkeitsänderungen bzw. Intensitätsänderungen auf der Detektionsfläche erkannt werden, indem mit dem erfindungsgemäßen Messansatz die Summe oder der Durchschnitt der betragsmäßigen Helligkeitsänderungen, die bei konstanter Beleuchtungsstärke durch dynamische Phänomene in der Probe erzeugt werden, flächenkumulativ, d. h. über die ganze Detektionsfläche gemessen werden. Dadurch wird vermieden, dass sich positive oder negative Einzelmessungen aufheben. Ferner wird die Sensitivität der Messung erhöht.

Unter einer optischen Weitfeld-Beleuchtungseinrichtung wird eine nicht-scannende Beleuchtungseinrichtung verstanden, d. h. eine Beleuchtungseinrichtung, mittels derer die ganze Probe ohne Einsatz einer Optik zur Fokussierung auf einzelne Bildebenen innerhalb der Probe bzw. ohne Einsatz einer Optik zum sukzessiven Abtasten des Probenvolumens beleuchtet werden kann. Bei einer solchen Weitfeld-Beleuchtungseinrichtung werden von dem Detektor alle Signalbeiträge aus dem gesamten Probenvolumen simultan erfasst.

Ein weiterer Vorteil des erfindungsgemäßen Verfahrens ist daher, dass das Verfahren unter Verwendung vergleichsweise einfacher optischer Elemente durchgeführt werden kann und auf Optiken zum sukzessiven Abtasten des Probenvolumens verzichtet werden kann. Daher kann die Vorrichtung zur Durchführung des Verfahrens kostengünstig und baulich kompakt ausgeführt sein. Ebenso weist das Verfahren eine nur geringe Störungsempfindlichkeit gegenüber Dejustagen auf.

Aufgrund der einfachen Auswertbarkeit und hohen Sensitivität des Messsignals und des baulich kompakten Aufbaus ist das Verfahren besonders für die parallele Überwachung einer Vielzahl von Proben geeignet und lässt sich prozesseffizient in Screening-Umgebungen bzw. in automatisierte Hochdurchsatzverfahren, z. B. High-Throughput Screening-Verfahren, integrieren.

Gemäß einer bevorzugten Ausgestaltungsform wird die Ableitung nach der Zeit und die anschließende Gleichrichtung der Detektionssignale einzelner Detektionsbereiche mittels einer integrierten Schaltung durchgeführt. Die integrierte Schaltung kann beispielsweise eine CMOS-Schaltung sein (Schaltung basierend auf einem sich ergänzenden Metall-Oxid-Halbleiter, engl. complementary metal-oxide-semiconductor). Dies ermöglicht eine schnelle Verarbeitung der Messsignale und eine kostengünstige sowie baulich kompakte Ausführung des Detektors.

Eine Bewegung in der Probe und/oder eine Bewegung eines Probenbestandteils der Probe kann beispielsweise detektiert werden, falls ein Wert des Ausgabesignals des Detektors einen vorbestimmten Schwellenwert übersteigt. Bei Muskelzellen kann eine Bewegung in der Probe auch detektiert werden, falls das Ausgabesignal eine Periodizität aufweist.

Gemäß einer besonders bevorzugten Ausgestaltungsform wird eine Bewegung in der Probe anhand einer Veränderung des Beugungsmusters erkannt. Gemäß dieser Ausgestaltungsform umfasst die optische Weitfeld-Beleuchtungseinrichtung eine Lichtstrahlenquelle, die kohärentes Licht erzeugt, wobei zumindest ein Teil eines Beugungsmusters, das von durch die Probe gebeugtem Licht der Lichtstrahlenquelle erzeugt wird, auf die Detektionsfläche fällt.

Bei einem Beugungsmuster enthält jeder Punkt des Musters Signalbeiträge der gesamten Probe. Es ist somit prinzipiell ausreichend, einen Teilbereich des von der Probe erzeugten Beugungsmusters mit dem Detektor zu erfassen. Eine Bewegung innerhalb der Probe erzeugt eine Änderung des Beugungsmusters, beispielsweise eines Speckle-Musters, jedoch bleibt die Gesamthelligkeit des sich ändernden Beugungsmusters zeitlich in etwa konstant. Durch den erfindungsgemäßen Messansatz, die Detektionsfläche in kleinere Detektionsbereiche zu unterteilen und die zeitliche Änderung der Messkurven gleichzurichten, bevor diese addiert oder gemittelt werden, wird verhindert, dass sich die von der Bewegung in der Probe erzeugten Änderungen ausmitteln.

Besonders vorteilhaft ist eine Variante, bei der das ganze Beugungsmuster auf die Detektionsfläche abgebildet wird. Hierdurch wird die Sensitivität der Bewegungserkennung erhöht. Zudem führt die Nutzung des gesamten Musters zu einer drastisch verbesserten Reproduzierbarkeit der Messungen (im Vergleich zur Messung eines kleinen Musterausschnitts) und zu einer gewissen quantitativen Vergleichbarkeit zwischen gleichartigen Proben.

Neben der beispielhaft hervorgehobenen Ausgestaltungform, bei der eine Bewegung in der Probe anhand einer Veränderung des erzeugten Beugungsmusters erkannt wird, kann die Weitfeld-Beleuchtungseinrichtung auch ausgebildet sein, ein Bild der Probe auf die Detektionsfläche abzubilden. Gemäß dieser alternativen Ausgestaltungsform umfasst die optische Weitfeld-Beleuchtungseinrichtung eine Lichtstrahlenquelle, die kohärentes oder nicht kohärentes Licht erzeugen kann, und eine Optik, die ausgebildet ist, einen Abbildungsstrahlengang zu erzeugen, um die Probe auf die Detektionsfläche des Detektors abzubilden.

Hier können unterschiedlichste Weitfeld-Beleuchtungs- bzw. Abbildungsansätze verwendet werden. Beispielsweise kann die optische Weitfeld-Beleuchtungseinrichtung ein Durchlichtmikroskop, ein Dunkelfeld-Mikroskop oder ein Weitfeld-Fluoreszenz-Mikroskop sein. Je nach Art der Weitfeld-Beleuchtungseinrichtung bzw. Positionierung des Detektors zur Probe kann es sich bei der vom Detektor erfassten und von der Probe kommenden Strahlung um Strahlung, insbesondere Licht, einer Strahlungsquelle der Weitfeld-Beleuchtungseinrichtung handeln, die an einer beliebigen Stelle innerhalb der Probe durch eine Wechselwirkung mit der Probe in ihrer Strahlrichtung und/oder ihrem Polarisationszustand verändert wird. Es kann sich auch um Durchlicht oder um Fluoreszenzstrahlung handeln.

Die Weitfeld-Beleuchtungs-Einrichtung kann eine auf der Beleuchtungsseite angeordnete Beleuchtungsoptik umfassen, mittels derer die Strahlung der Lichtstrahlenquelle auf die ganze Probe geleitet wird, um die Probe vollständig und möglichst gleichmäßig zu beleuchten. Die Optik kann ferner eine Detektionsoptik umfassen, mittels derer das von der Probe ausgesandte Licht, das durch eine Wechselwirkung mit der Probe in ihrer Strahlrichtung, ihrem Polarisationszustand und/oder ihrem Beugungsmuster verändert wird, auf eine Detektionsfläche des Detektors geleitet wird. Diese funktionalen Eigenschaften der Beleuchtungsoptik und/oder der Detektionsoptik können dabei unter Verwendung eines oder mehrerer zweckmäßig angeordneter und ausgestalteter bekannter optischer Bauelemente und Komponenten, wie z. B. Filter, Linsen, Blenden, refraktiver Elemente etc. realisiert werden.

Das Verfahren kann ferner das Bereitstellen einer Aufnahme für die Probe umfassen. Die Aufnahme zur Halterung der Probe kann eine Trägermatrix, vorzugsweise ein Biopolymer umfassend. Ferner kann die Aufnahme der Probe als eine sich in einem hängenden Tropfen befindliche Trägermatrix, vorzugsweise ein Biopolymer wie z. B. Alginat, ausgeführt sein. Für die Anwendung des Verfahrens in Screening-Umgebungen kann die Aufnahme für die Probe eine Kavität einer Multiwell-Platte (Mikrotiterplatte) sein. Weiterhin kann die Aufnahme eine Kavität einer Multiwell-Platte sein, die zur Ausbildung eines hängenden Tropfens an den einzelnen Kavitäten ausgebildet ist (sog. Hängender-Tropfen-Multiwell-Platte). Solche Hängender-Tropfen-Multiwell-Platten werden beispielsweise von der Firma Insphero AG, CH-8952 Schlieren unter der Bezeichnung "GravityPLUS^{™} 3D Culture and Assay Platform" angeboten. Auch die Patentschrift EP 2 342 317 B1 offenbart eine solche Platte.

Vorstehend wurde bereits erwähnt, dass sich das Verfahren besonders für die parallele Überwachung einer Vielzahl von Proben eignet, z. B. von Proben, die im Rahmen von automatisierten Hochdurchsatzverfahren untersucht werden sollen. Eine vorteilhafte Weiterbildung des Verfahrens sieht daher vor, dass mit diesem eine parallele optische Detektion von Bewegungen in mehreren voneinander getrennten biologischen Proben oder von Bewegungen von Probenbestandteilen in mehreren voneinander getrennten biologischen Proben durchgeführt wird. Gemäß dieser Variante kann die Aufnahme für die Proben eine Multiwell-Platte oder eine Hängender-Tropfen-Multiwell-Platte sein, die eine Mehrzahl von in Reihen und Spalten angeordnete Kavitäten zur Aufnahme der Proben aufweist. Ferner sind mehrere Detektoren, wie in diesem Dokument beschrieben, in Form eines Detektoranordnung vorgesehen, wobei je ein Detektor der Detektoranordnung einer Kavität zugeordnet ist. Hierbei entspricht vorzugsweise ein Rasterabstand der einzelnen Detektoren einem Rasterabstand der Kavitäten der Multiwell-Platte.

Der Durchmesser der Detektionsfläche des Detektors kann kleiner gleich 9 mm sein. Dies ist besonders vorteilhaft, falls der Detektor zur Bewegungsdetektion von in Multi-Well-Platen gelagerten Proben genutzt wird, insbesondere von in 96-WellPlatten gelagerten Proben.

Bei einer parallelen optischen Detektion einer Bewegung in mehreren voneinander getrennten biologischen Proben, die in jeweils einer Kavität oder einem hängenden Tropfen einer Multiwell-Platte gelagert sind, kann die optische Weitfeld-Beleuchtungseinrichtung ausgebildet sein, die einzelnen Kavitäten zu beleuchten. Falls kohärentes Licht benötigt wird, z. B. für die Ausführungsform, bei der ein Beugungsmuster erfasst wird, kann die optische Weitfeld-Beleuchtungseinrichtung als ein Laserdiodenarray ausgeführt sein, wobei ein Rasterabstand der einzelnen Laserdioden dem Rasterabstand der Kavitäten der Multiwell-Platte entspricht. Ein Laserdiodenarray stellt eine platzsparende und energieeffiziente Beleuchtungsquelle dar.

Anstatt eines Laserdiodenarrays als Lichtstrahlenquelle kann die Lichtstrahlenquelle als herkömmliche Lichtquelle (Laser, Bogenlampe etc.) ausgeführt sein, wobei das Licht der Lichtstrahlenquelle zur Beleuchtung der Proben über ein Lichtfaserbündel in die einzelnen Kavitäten, die die Proben enthalten, eingekoppelt wird. Jede Lichtfaser ist dabei einer Kavität zugeordnet. Dies bietet den Vorteil, dass die Lichtquelle ausreichend beabstandet zur Probe betrieben werden kann, um eine zu starke Wärmeentwicklung in Probennähe zu vermeiden.

Ferner besteht gemäß einer weiteren alternativen Variante die Möglichkeit, die Multiwell-Platte mit einer Lichtstrahlenquelle flächig zu beleuchten, was eine einfache Umsetzungsvariante darstellt, jedoch Energieeffizienznachteile hat, da die Lichtstrahlenquelle entsprechend leistungsfähig sein muss. Vorteilhaft ist es hierbei, wenn das Licht über eine zweckmäßig ausgeführte Kondensoroptik und ggf. winkelabhängige Durchlassfilter parallelisiert und gezielt auf die Kavitäten geleitet wird.

Gemäß einem zweiten Gesichtspunkt der Erfindung wird eine Vorrichtung zur kontaktfreien In-Vitro-Detektion einer Bewegung in einer biologischen Probe und/oder einer Bewegung eines Probenbestandteils der biologischen Probe bereitgestellt.

Der Durchmesser der biologischen Probe kann beispielsweise mindestens 50 µm betragen. Die Vorrichtung umfasst eine optische Weitfeld-Beleuchtungseinrichtung zur Beleuchtung der Probe, die ausgebildet ist, die ganze Probe zu beleuchten. Die Vorrichtung umfasst ferner einen Detektor zur Erfassung von der Probe kommender Strahlung, wobei der Detektor eine Detektionsfläche aufweist, die in mehrere Detektionsbereiche unterteilt ist. Der Detektor ist ausgebildet, Detektionssignale einzelner Detektionsbereiche nach der Zeit abzuleiten, anschließend gleichzurichten, vorzugsweise durch Betragsbildung oder Quadrierung, und die abgeleiteten und gleichgerichteten Detektionssignale aller Detektionsbereiche zu summieren oder zu mitteln und dann als Ausgabesignal bereitzustellen.

Gemäß einer bevorzugten Ausführungsform der Erfindung weist der Detektor eine integrierte Schaltung, vorzugsweise eine CMOS-Schaltung, auf, die ausgebildet ist, die Ableitung nach der Zeit und die anschließende Gleichrichtung der Detektionssignale einzelner Detektionsbereiche durchzuführen.

Der Detektor kann ein einkanaliges Signal ausgeben und/oder ein in Bezug auf die Detektionsfläche nicht ortsaufgelöstes Signal ausgeben.

Gemäß einem dritten Gesichtspunkt der Erfindung wird ein Detektor zur Erfassung optischer Strahlung bereitgestellt, mit einer Detektionsfläche, die in mehrere Detektionsbereiche (4a) unterteilt ist, wobei der Detektor ausgebildet ist, Detektionssignale einzelner Detektionsbereiche nach der Zeit abzuleiten, anschließend gleichzurichten, vorzugsweise durch Betragsbildung oder Quadrierung, und die abgeleiteten und gleichgerichteten Detektionssignale aller Detektionsbereiche zu summieren oder zu mitteln und dann als Ausgabesignal bereitzustellen.

Zur Vermeidung von Wiederholungen sollen rein vorrichtungsgemäß offenbarte Merkmale auch als verfahrensgemäß offenbart gelten und beanspruchbar sein und umgekehrt. Die vorgenannten Aspekte und erfindungsgemäßen Merkmale, insbesondere im Hinblick auf die Ausbildung des Detektors oder der optischen Weitfeld-Beleuchtungseinrichtung, die im Zusammenhang mit dem Verfahren beschrieben wurden, gelten somit beispielsweise auch für die Vorrichtung.

Die zuvor beschriebenen bevorzugten Ausführungsformen und Merkmale der Erfindung sind beliebig miteinander kombinierbar. Weitere Einzelheiten und Vorteile der Erfindung werden im Folgenden unter Bezug auf die beigefügten Zeichnungen beschrieben. Es zeigen:
- Figur 1: eine schematische Darstellung eines Verfahrens und einer Vorrichtung gemäß einer Ausführungsform der Erfindung;
- Figur 2: eine Darstellung eines auf die Detektorfläche abgebildeten Speckle-Musters;
- Figur 3: eine schematische Illustration der Datenverarbeitung des Detektors gemäß einer Ausführungsform der Erfindung;
- Figur 4: den Effekt der Differenzierung und Gleichrichtung der Messwerte einzelner Pixel; und
- Figur 5: eine schematische Darstellung eines Verfahrens und einer Vorrichtung gemäß einer weiteren Ausführungsform der Erfindung.

Gleiche Teile sind in den Figuren mit denselben Bezugszeichen versehen und werden nicht gesondert beschrieben.

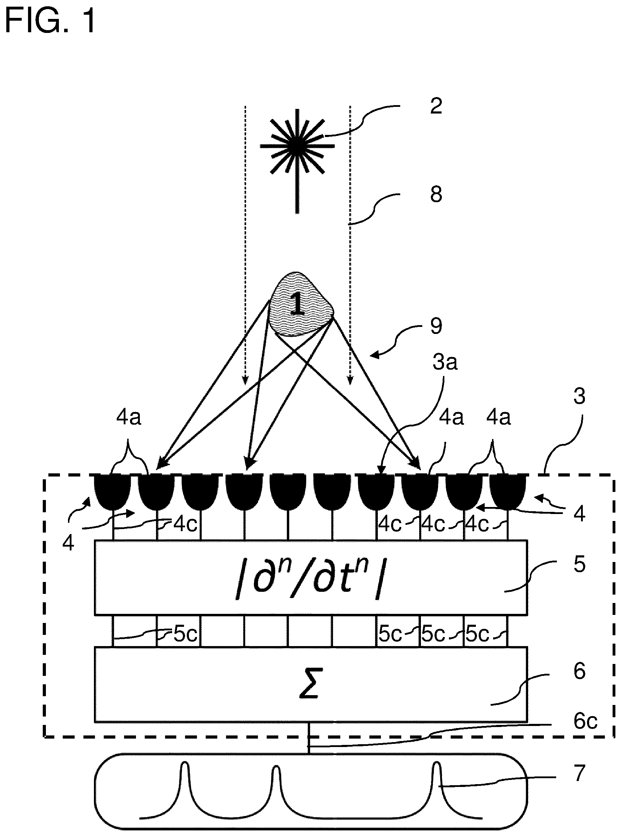

Figur 1 zeigt eine schematische Darstellung eines Verfahrens und einer Vorrichtung gemäß einer Ausführungsform der Erfindung.

Zur Durchführung des Verfahrens wird eine Vorrichtung zur optischen In-Vitro-Detektion einer Bewegung in einer biologischen Probe 1 bereitgestellt. Bei der biologischen Probe 1 kann es sich um einen Zellhaufen aus Muskelgewebe handeln.

Die Vorrichtung umfasst eine Aufnahme (nicht dargestellt) für die Probe 1, eine optische Weitfeld-Beleuchtungseinrichtung 2 und einen Detektor 3.

Die Aufnahme ist nicht auf eine bestimmte Art von Aufnahmen beschränkt, sondern kann je nach Anwendungszweck und Art der Probe zweckmäßig, z. B. als Träger, Trägerplatte, als Gefäß, als Kavität einer Multiwellplatte oder als Trägermatrix in Form eines Biopolymers, z. B. Alginats, auf dem die Probe gezüchtet wird, ausgeführt sein.

Bei dem in Figur 1 gezeigten Ausführungsbeispiel ist die optische Weitfeld-Beleuchtungseinrichtung eine Strahlungsquelle, die kohärentes Licht erzeugt (Laser). Mittels derer von der Strahlungsquelle 2 erzeugten kohärenten Strahlung 8 wird die ganze Probe 1 beleuchtet. Hierzu kann es zweckmäßig sein, eine Beleuchtungsoptik (nicht dargestellt) vorzusehen, mittels der die Strahlung der Lichtstrahlenquelle 2 auf die ganze Probe 1 geleitet wird, um die Probe vollständig und möglichst gleichmäßig zu beleuchten. Diese funktionale Eigenschaft der Optik kann dabei unter Verwendung eines oder mehrerer zweckmäßig angeordneter und ausgestalteter bekannter optischer Bauelemente und Komponenten, wie z. B. Filter, Linsen, Blenden, refraktiver Elemente etc., realisiert werden.

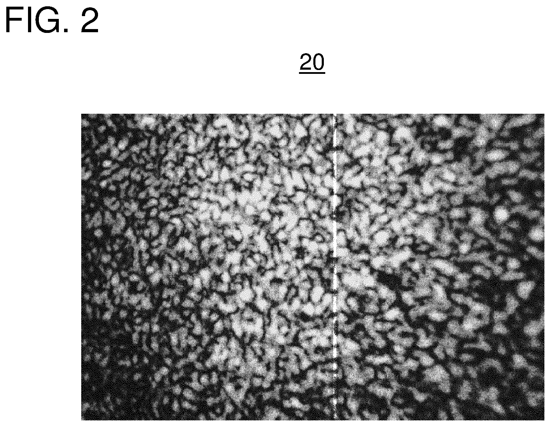

Das an der Probe 1 gebeugte Licht der Lichtstrahlenquelle 2 erzeugt ein Beugungsmuster mit einem Zentrum hoher Intensität und einem Randbereich niedriger Intensität, z. B. in Form eines Speckle-Musters 20, das beispielhaft in Figur 2 gezeigt ist.

Die Vorrichtung umfasst ferner einen optischen Detektor 3 zur Erfassung des Beugungsmusters 20. Der Detektor 3 ist so angeordnet, dass das Beugungsmuster 20, das von durch die Probe 1 gebeugtem Licht 9 der Lichtstrahlenquelle 2 erzeugt wird, auf die Detektionsfläche 3a des Detektors 3 abgebildet wird.

Hierbei ist die Detektionsfläche als zweidimensionale Pixelarrayfläche ausgebildet, so dass die Detektionsfläche 3a durch die einzelnen Pixel 4 in mehrere Detektionsbereiche 4a unterteilt ist. Die Detektionsfläche ist in mehrere Pixel 4, z. B. in 10*10 Pixel 4 oder mehr unterteilt, was in der Prinzipdarstellung der Figur 1 nicht dargestellt ist. Jeder Pixel misst somit einen Ausschnitt des Speckel-Musters 20.

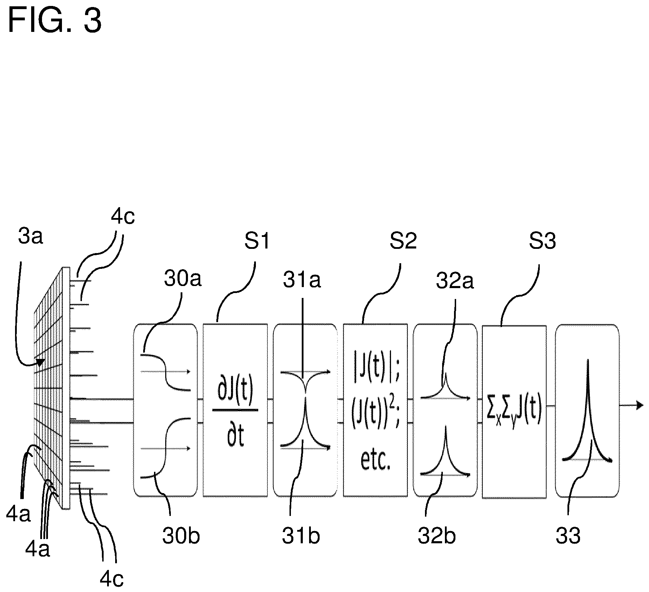

Die Datenverarbeitung basierend auf der gemessenen Lichtintensität der einzelnen Pixel 4 wird nachfolgend auch unter Bezugnahme auf Figur 3 beschrieben.

Figur 3 zeigt in einer perspektivischen Darstellung die Detektionsfläche 3a, die in einzelne Pixel 4a unterteilt ist. Jeder Pixel 4 gibt den Zeitverlauf der auf der jeweiligen Pixelfläche (Detektionsbereich) 4a gemessenen Lichtintensität in Form eines Detektionssignals 4c aus. Mit dem Bezugszeichen 30a ist lediglich beispielhaft ein solcher zeitlicher Verlauf eines ersten Pixels gemessen, der eine Abnahme der Lichtintensität misst, was durch den abfallenden Kurvenverlauf dargestellt ist. Zeitgleich kann ein zweiter Pixel eine Zunahme der Lichtintensität messen, was mit dem Bezugszeichen 30b dargestellt ist.

Eine solche zeitliche Veränderung resultiert aus einer Bewegung innerhalb der Probe bzw. aus einer Bewegung eines Probenbestandteils, wodurch das von der Probe erzeugte BeugungsMuster 20 geändert wird.

In einem ersten Schritt S1 werden die gemessenen Detektionssignale 4c der einzelnen Pixel 4 nach der Zeit abgeleitet. Das Ergebnis dieser Differenzierung-Operation ist beispielhaft durch die Kurven 31a bzw. 31b dargestellt. Anschließend erfolgt in Schritt S2 eine Gleichrichtung der einzelnen Ergebnisse, d. h., das differenzierte Messergebnis jedes Pixels 4 wird beispielsweise durch eine Betragsbildung oder eine Quadrierung gleichgerichtet. Das Ergebnis dieser Gleichrichtung ist beispielhaft durch die Kurven 32a bzw. 32b dargestellt.

In einem dritten Schritt S3 werden die abgeleiteten und gleichgerichteten Detektionssignale aller Pixel 4 summiert (oder alternativ gemittelt) und dann als Ausgabesignal 6c des Detektors 3 bereitgestellt. Das Ergebnis dieser Summierung ist beispielhaft durch die Kurve 33 dargestellt.

Vorliegend erfolgt die zeitliche Ableitung und anschließende Gleichrichtung der Messdaten der einzelnen Pixel durch eine integrierte CMOS-Schaltung 5 des Detektors 3. Diese CMOS-Schaltung gibt somit für jeden Pixel ein differenziertes und gleichgerichtetes Signal 5c aus. Diese Ausgangsdaten der CMOS-Schaltung werden nachfolgend in einem Summierer 6 addiert.

Der Detektor 3 erscheint somit nach außen als Detektor, der nur einen Datenausgang 6c aufweist, über den ein nicht ortsaufgelöstes Signal/Daten 7 ausgegeben wird. Das in Bezug auf die Detektorfläche 3a nicht ortaufgelöste Signal misst flächenkumulativ, d. h. über die ganze Detektionsfläche, die Summe (oder den Durchschnitt) an betragsmäßigen Helligkeitsänderungen, die bei konstanter Beleuchtungsstärke durch dynamische Phänomene in der Probe erzeugt werden.

Die Bewegungen in der Probe führen dann zu den Peaks im Ausgabesignal 7, die leicht erkannt werden können.

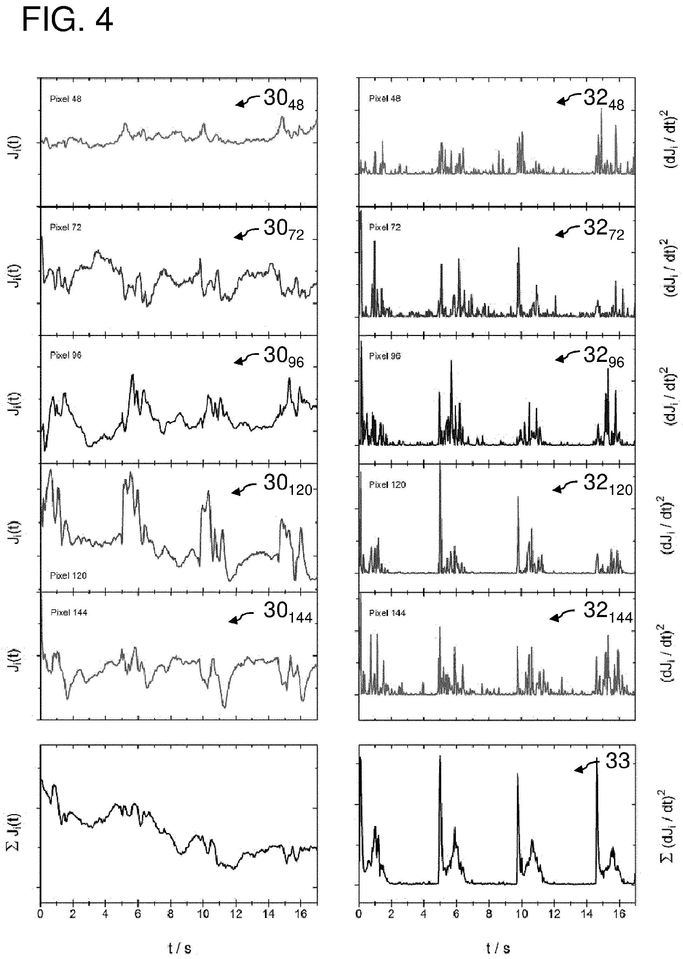

Zur Verdeutlichung der Vorteile des erfindungsgemäßen Ansatzes illustriert Figur 4 den Effekt der Differenzierung und Gleichrichtung der Messwerte zufällig ausgewählter Bildbereiche (vereinfacht "Pixel" genannt) 4 der Detektionsfläche 3a, hier der Pixel 48, 72, 96, 120, 144. Hier wurde zur Demonstration des Verfahrens das Video eines an einem Herzmuskel-Gewebemodell aufgenommenen Speckle-Musters per Videosoftware in 192 Bildsegmente ("Pixel") zerlegt und die mittlere Helligkeit jeden Segmentes zeitabhängig aufgezeichnet (Jᵢ(t)). Mit Hilfe einer Datenanalysesoftware wurden diese Jᵢ(t) abgeleitet und quadriert, anschließend diese bearbeiteten Datensätze summiert (Σ(∂Jᵢ/∂t)²)

Die fünf oberen Diagramme in der linken Spalte der Figur 4 zeigen den zeitlichen Verlauf 30 der gemessenen Lichtintensität, der jeweils durch die Pixel 48, 72, 96, 120, 144 des Detektors 3 gemessen wird. Addiert man die Messwerte aller Bildsegmente, ergibt sich der im linken unteren Diagramm der Figur 4 gezeigte zeitliche Verlauf. Eine Bewegung in der Probe ist nicht zuverlässig detektierbar.

Die fünf oberen Diagramme in der rechten Spalte der Figur 4 zeigen den differenzierten und anschließend gleichgerichteten zeitlichen Verlauf 32 der gemessenen Lichtintensität der einzelnen Pixel 48, 72, 96, 120, 144.

Addiert man die bearbeiteten Werte aller Bildsegmente, ergibt sich der im rechten unteren Diagramm der Figur 4 gezeigte zeitliche Verlauf 33. Die Bewegung in der Probe ist nun gut anhand der einzelnen Peaks des Verlaufs 33 detektierbar.

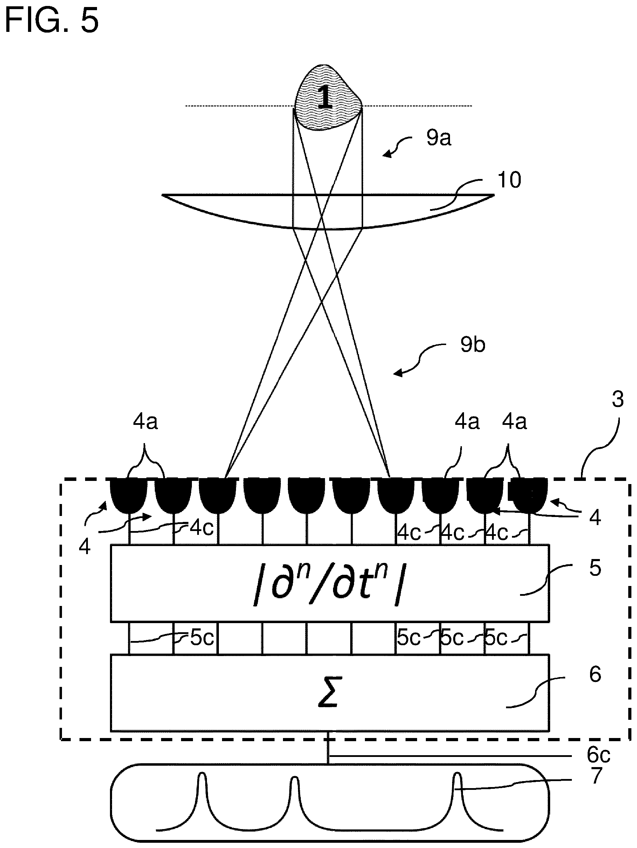

Figur 5 zeigt eine schematische Darstellung eines Verfahrens und einer Vorrichtung gemäß einer weiteren Ausführungsform der Erfindung. Hierbei entsprechen Komponenten mit gleichen Bezugszeichen den Komponenten der Figur 1 und werden nicht gesondert beschrieben.

Die ganze biologische Probe 1 wird wiederum mittels einer optischen Weitfeld-Beleuchtungseinrichtung, d. h. ohne Einsatz einer Optik zur Fokussierung auf einzelne Bildebenen innerhalb der Probe bzw. ohne Einsatz einer Optik zum sukzessiven Abtasten des Probenvolumens, beleuchtet. Eine Besonderheit dieser Ausführungsform liegt darin, dass die Strahlungsquelle der optischen Weitfeld-Beleuchtungseinrichtung kein kohärentes Licht erzeugt. Folglich wird kein detektierbares Beugungsmuster von der Probe 1 erzeugt. Stattdessen ist eine Optik 10, z. B. in Form einer konvexen Linse, der optischen Weitfeld-Beleuchtungseinrichtung vorgesehen, mittels derer ein Abbildungsstrahlengang 9a, 9b erzeugt wird, um die eine Fokalebene der Probe 1 auf die Detektionsfläche 3a des Detektors 3 abzubilden. Die optische Weitfeld-Beleuchtungseinrichtung kann beispielsweise ein Durchlichtmikroskop, ein Dunkelfeld-Mikroskop oder ein Weitfeld-Fluoreszenz-Mikroskop sein. Die Verarbeitung der Messdaten erfolgt analog wie in dem vorstehend beschriebenen Ausführungsbeispiel. Die Ausführung mit abbildender Optik ist somit auch zur Überwachung räumlicher Proben geeignet, jedoch ist die auf Beugungsmustern basierende Ausführung als vorteilhafter anzustehen.

Obwohl die Erfindung unter Bezugnahme auf bestimmte Ausführungsbeispiele beschrieben worden ist, ist es für einen Fachmann ersichtlich, dass verschiedene Änderungen ausgeführt werden können und Äquivalente als Ersatz verwendet werden können, ohne den Bereich der Erfindung zu verlassen. Zusätzlich können viele Modifikationen ausgeführt werden, ohne den zugehörigen Bereich zu verlassen. Folglich soll die Erfindung nicht auf die offenbarten Ausführungsbeispiele begrenzt sein, sondern soll alle Ausführungsbeispiele umfassen, die in den Bereich der beigefügten Patentansprüche fallen. Insbesondere beansprucht die Erfindung auch Schutz für den Gegenstand und die Merkmale der Unteransprüche unabhängig von den in Bezug genommenen Ansprüchen.

Die Offenbarung der Erfindung umfasst ferner folgende Aspekte:
Gemäß einem ersten Aspekt, ein Verfahren zur optischen In-Vitro-Detektion einer Bewegung in einer biologischen Probe (1) und/oder einer Bewegung eines Probenbestandteils der biologischen Probe (1), umfassend die Schritte:
a) Bereitstellen
a1) einer optischen Weitfeld-Beleuchtungseinrichtung zur Beleuchtung der Probe, die ausgebildet ist, die ganze Probe (1) zu beleuchten, und
a2) eines Detektors (3) zur Erfassung von der biologischen Probe kommender Strahlung (9; 9a, 9b), wobei der Detektor 3) eine Detektionsfläche (3a) aufweist, die in mehrere Detektionsbereiche (4a) unterteilt ist und ausgebildet ist, Detektionssignale (4c) einzelner Detektionsbereiche (4a) nach der Zeit abzuleiten (S1), anschließend gleichzurichten (S2), vorzugsweise durch Betragsbildung oder Quadrierung, und die abgeleiteten und gleichgerichteten Detektionssignale aller Detektionsbereiche zu summieren oder zu mitteln (S3) und dann als Ausgabesignal (6c) bereitzustellen.
b) Beleuchten der biologischen Probe (1) mit der Weit-feld-Beleuchtungseinrichtung; und
c) Detektieren einer Bewegung in der biologischen Probe (1) in Abhängigkeit von dem Ausgabesignal (6c) des Detektors (3).

Bei einem optionalen Aspekt des Verfahrens kann die Ableitung nach der Zeit (S1) und anschließende Gleichrichtung (S2) der Detektionssignale einzelner Detektionsbereiche mittels einer integrierten Schaltung (5), vorzugsweise einer CMOS-Schaltung, im Detektor (3) durchgeführt werden.

Weitere optionale Aspekte des Verfahrens sehen vor, a) dass der Detektor (3) ein einkanaliges Signal (33) ausgibt; und/oder b) dass der Detektor ein in Bezug auf die Detektions-fläche (3a) nicht ortsaufgelöstes Signal (33) oder nicht ortsaufgelöste Daten ausgibt; und/oder c) dass der Detektor (3) ein nicht bildgebender Detektor ist.

Bei einem optionalen Aspekt des Verfahrens kann die optische Weitfeld-Beleuchtungseinrichtung eine Lichtstrahlenquelle (2) umfassen, die kohärentes Licht erzeugt, wobei zumindest ein Teil eines Beugungsmusters (20), das von durch die Probe (1) gebeugtem Licht (9) der Lichtstrahlenquelle (2) erzeugt wird, auf die Detektionsfläche (3a) des Detektors (3) abgebildet wird.

Bei einem alternativen optionalen Aspekt des Verfahrens kann die optische Weitfeld-Beleuchtungseinrichtung eine Lichtstrahlenquelle und eine Optik (10) umfassen, wobei die Optik (10) ausgebildet ist, einen Abbildungsstrahlengang (9a, 9b) zu erzeugen, um die Probe (1) auf die Detektionsfläche (3a) des Detektors (3) abzubilden.

Hierbei kann die optische Weitfeld-Beleuchtungseinrichtung ein Durchlichtmikroskop, ein Dunkelfeld-Mikroskop oder ein Weitfeld-Fluoreszenz-Mikroskop sein.

Bei einem optionalen Aspekt des Verfahrens kann eine Bewegung in der Probe (1) und/oder eine Bewegung eines Probenbestandteils der Probe (1) detektiert werden, falls ein Wert des Ausgabesignals (33) des Detektors einen vorbestimmten Schwellenwert übersteigt und/oder falls das Ausgabesignal (33) eine Periodizität aufweist.

Weitere optionale Aspekte des Verfahrens sehen vor, dass die biologische Probe (1) a) eine dreidimensionale Zell- und/oder Gewebekultur oder ein Zellhaufen ist; oder b) eine Probe aus freischwimmenden Mikroorganismen, beispielsweise Spermien, ist.

Weitere optionale Aspekte des Verfahrens sehen vor, dass eine Aufnahme zur Halterung der Probe (1) a) eine Trägermatrix, vorzugsweise ein Biopolymer (15), umfasst; und/oder b)einen hängenden Tropfen umfasst; und/oder c) eine Kavität einer Multiwell-Platte oder eine Hängender-Tropfen-Multiwell-Platte ist.

Eine weitere Ausführungsform sieht ein Verfahren vor, bei dem eine parallele optische Detektion von jeweils einer Bewegung in mehreren voneinander getrennten biologischen Proben oder von jeweils einer Bewegung eines Probenbestandteils in mehreren voneinander getrennten biologischen Proben durchgeführt wird, wobei eine Aufnahme für die Proben eine Multiwell-Platte oder eine Hängender-Tropfen-Multiwell-Platte ist, die eine Mehrzahl von in Reihen und Spalten angeordnete Kavitäten zur Aufnahme der Proben aufweist, und wobei mehrere Detektoren (3) in Form eines Detektor-arrays vorgesehen sind, wobei je ein Detektor des Detektor¬arrays einer Kavität zugeordnet ist.

Bei einem optionalen Aspekt des Verfahrens kann ein Durchmesser der Detektionsfläche (3a) des Detektors (3) kleiner gleich 9 mm sein.

Weitere optionale Aspekte des Verfahrens sehen vor, a) dass die biologische Probe (1) eine Mehrzahl von Zellen umfasst; und/oder b) dass ein Durchmesser der biologischen Proben in allen Raumrichtungen mindestens 50 Mikrometer beträgt; und/oder c) dass ein Durchmesser der biologischen Probe größer ist als eine von der optischen Weitfeldbeleuchtungseinrichtung erreichbare, beugungsbegrenzte Auflösung.

Gemäß einem zweiten Aspekt umfasst die Offenbarung eine Vorrichtung zur kontaktfreien In-Vitro-Detektion einer Bewegung in einer biologischen Probe (1) und/oder einer Bewegung eines Probenbestandteils der biologischen Probe (1). Die Vorrichtung umfasst eine optische Weitfeld-Beleuchtungseinrichtung zur Beleuchtung der Probe (1), die ausgebildet ist, die ganze Probe (1) zu beleuchten, und einen Detektor (3) zur Erfassung von der Probe (1) kommender Strahlung (9; 9a, 9b), wobei der Detektor (3) eine Detektionsfläche (3a) aufweist, die in mehrere Detektionsbereiche (4a) unterteilt ist und ausgebildet ist (5,6), Detektionssignale (4c) einzelner Detektionsbereiche (4a) nach der Zeit abzuleiten, anschließend gleichzurichten, vorzugsweise durch Betragsbildung oder Quadrierung, und die abgeleiteten und gleichgerichteten Detektionssignale aller Detektionsbereiche zu summieren oder zu mitteln und dann als Ausgabesignal (33) bereitzustellen.

Bei einer Ausführungsform der Vorrichtung kann der Detektor eine integrierte Schaltung (5), vorzugsweise eine CMOS-Schaltung, aufweisen, die ausgebildet ist, die Ableitung nach der Zeit und die anschließende Gleichrichtung der Detektionssignale (4c) einzelner Detektionsbereiche (4a) durchzuführen.

Weitere optionale Aspekte der Vorrichtung sehen vor, a) dass der Detektor (3) ein einkanaliges Signal (33) ausgibt; und/oder b) dass der Detektor (3) ein in Bezug auf die Detektionsfläche nicht ortsaufgelöstes Signal (33) ausgibt; und/oder c) dass die Detektionsfläche (3a) in mindestens 100 bzw. 10*10 mehrzeilig nebeneinander angeordnete Pixel (4) unterteilt ist.

Gemäß einem dritten Aspekt umfasst die Offenbarung einen Detektor (3) zur Erfassung optischer Strahlung, mit einer Detektionsfläche (3a), die in mehrere Detektionsbereiche (4a) unterteilt ist, wobei der Detektor (3) ausgebildet ist (5, 6), Detektionssignale (4c) einzelner Detektionsbereiche (4c) nach der Zeit abzuleiten, anschließend gleichzurichten, vorzugsweise durch Betragsbildung oder Quadrierung, und die abgeleiteten und gleichgerichteten Detektionssignale aller Detektionsbereiche zu summieren oder zu mitteln und dann als Ausgabesignal (33) bereitzustellen.

## Patentansprüche

1. Detektor (3) zur Erfassung optischer Strahlung, mit einer Detektionsfläche (3a), die in mehrere Detektionsbereiche (4a) unterteilt ist, wobei der Detektor (3) ausgebildet ist (5, 6), Detektionssignale (4c) einzelner Detektionsbereiche (4c) nach der Zeit abzuleiten, anschließend gleichzurichten, vorzugsweise durch Betragsbildung oder Quadrierung, und die abgeleiteten und gleichgerichteten Detektionssignale aller Detektionsbereiche zu summieren oder zu mitteln und dann als Ausgabesignal (33) bereitzustellen; und wobei der Detektor (3) ein nicht bildgebender Detektor ist.

2. Detektor (3) nach Anspruch 1, **dadurch gekennzeichnet, dass** der Detektor eine integrierte Schaltung (5), vorzugsweise eine CMOS-Schaltung, aufweist, die ausgebildet ist, die Ableitung nach der Zeit und die anschließende Gleichrichtung der Detektionssignale (4c) einzelner Detektionsbereiche (4a) durchzuführen.

3. Detektor (3) nach einem der vorhergehenden Ansprüche, **dadurch gekennzeichnet, dass** der Detektor (3) ausgebildet ist, ein einkanaliges Signal (33) auszugeben.

4. Detektor (3) nach einem der vorhergehenden Ansprüche, **dadurch gekennzeichnet, dass** der Detektor (3) ausgebildet ist, ein in Bezug auf die Detektionsfläche (3a) nicht ortsaufgelöstes Signal (33) oder nicht ortsaufgelöste Daten auszugeben.

5. Detektor (3) nach einem der vorhergehenden Ansprüche, **dadurch gekennzeichnet, dass** ein Durchmesser der Detektionsfläche (3a) des Detektors (3) kleiner gleich 9 mm ist.

6. Detektor (3) nach einem der vorhergehenden Ansprüche, **dadurch gekennzeichnet, dass** die Detektionsfläche (3a) in mindestens 100 oder 10*10 mehrzeilig nebeneinander angeordnete Pixel (4) unterteilt ist.

7. Detektor (3) nach einem der vorhergehenden Ansprüche, **dadurch gekennzeichnet, dass** die Detektionsfläche (3a) in mindestens 10000 oder 100*100 mehrzeilig nebeneinander angeordnete Pixel (4) unterteilt ist.

## Claims

1. Detector (3) for detecting optical radiation with a detection area (3a) which is subdivided into a plurality of detection regions (4a), wherein the detector (3) is configured (5, 6) to form the derivative with respect to time of detection signals (4c) of individual detection regions (4c), subsequently to rectify them, preferably by absolute value generation or squaring, and to sum or to average the differentiated and rectified detection signals of all the detection regions and then to provide them as an output signal (33); and wherein the detector (3) is a non-image forming detector.

2. Detector (3) according to claim 1, **characterised in that** the detector (3) comprises an integrated circuit (5), preferably a CMOS circuit, that is configured to perform the derivative with respect to time and the subsequent rectification of the detection signals (4c) of individual detection regions (4a).

3. Detector (3) according to one of the preceding claims, **characterised in that** the detector (3) is configured to output a single-channel signal (33).

4. Detector (3) according to one of the preceding claims, **characterised in that** the detector (3) is configured to output a signal (33) that is not spatially resolved or data that is not spatially resolved with respect to the detection area (3a).

5. Detector (3) according to one of the preceding claims, **characterised in that** a diameter of the detection area (3a) of the detector (3) is smaller than or equal to 9 mm.

6. Detector (3) according to one of the preceding claims, **characterised in that** the detection area (3a) is subdivided into at least 100 or 10 x 10 multi-line adjacently arranged pixels (4).

7. Detector (3) according to one of the preceding claims, **characterised in that** the detection area (3a) is subdivided into at least 10000 or 100*100 multi-line adjacently arranged pixels (4).

## Revendications

1. Détecteur (3) destiné à la détection d'un rayonnement optique, comprenant une surface de détection (3a) qui est subdivisée en plusieurs zones de détection (4a), le détecteur (3) étant configuré (5, 6) pour effectuer la dérivation temporelle de signaux de détection (4c) de zones de détection individuelles (4a), puis les redresser, de préférence par formation de la valeur absolue ou par élévation au carré, et pour sommer ou moyenner les signaux de détection dérivés et redressés de toutes les zones de détection puis les fournir en tant que signal de sortie (33) ; et le détecteur (3) étant un détecteur non imageur.

2. Détecteur (3) selon la revendication 1, **caractérisé en ce que** le détecteur comprend un circuit intégré (5), de préférence un circuit CMOS, qui est configuré pour effectuer la dérivation temporelle et le redressement subséquent des signaux de détection (4c) de zones de détection individuelles (4a).

3. Détecteur (3) selon l'une quelconque des revendications précédentes, **caractérisé en ce que** le détecteur (3) est configuré pour délivrer un signal monocanal (33).

4. Détecteur (3) selon l'une quelconque des revendications précédentes, **caractérisé en ce que** le détecteur (3) est configuré pour délivrer un signal (33) non résolu spatialement par rapport à la surface de détection (3a) ou des données non résolues spatialement.

5. Détecteur (3) selon l'une quelconque des revendications précédentes, **caractérisé en ce qu'**un diamètre de la surface de détection (3a) du détecteur (3) est inférieur ou égal à 9 mm.

6. Détecteur (3) selon l'une quelconque des revendications précédentes, **caractérisé en ce que** la surface de détection (3a) est subdivisée en au moins 100 pixels (4), ou 10 × 10 pixels (4), disposés côte à côte sur plusieurs lignes.

7. Détecteur (3) selon l'une quelconque des revendications précédentes, **caractérisé en ce que** la surface de détection (3a) est subdivisée en au moins 10 000 pixels (4), ou 100 × 100 pixels (4), disposés côte à côte sur plusieurs lignes.
